# EUROPEAN PATENT SPECIFICATION

(11) **EP 3 456 263 B1**
(45) Date of publication and mention of the grant of the patent: **10.03.2021**
(21) Application number: 17796329.5
(22) Date of filing: 08.05.2017
(51) Int. Cl.: A61B 8/00, A61B 8/08

(54) **PORTABLE ULTRASONIC DIAGNOSTIC DEVICE AND METHOD FOR OPERATING SAME**
TRAGBARE ULTRASCHALLDIAGNOSEVORRICHTUNG UND VERFAHREN ZUM BETRIEB DAVON
DISPOSITIF DE DIAGNOSTIC ULTRASONORE PORTATIF ET SON PROCÉDÉ DE FONCTIONNEMENT

(30) Priority: 10.05.2016 KR 20160057023
(43) Date of publication of application: 20.03.2019
(73) Proprietor: Healcerion Co., Ltd., Seoul 08376 (KR)
(72) Inventor: RYU, Jeong Won, Seoul 06768 (KR); KIM, Seung Hyun, Seoul 07061 (KR)
(74) Representative: Brann AB
(86) International application number: PCT/KR2017/004743
(87) International publication number: WO 2017/196029

(56) References cited:
- WO-A1-2015/084092
- JP-A- 2002 209 897
- JP-A- 2006 122 586
- JP-A- 2011 212 070
- KR-A- 20140 026 289
- KR-B1- 101 515 809
- US-A1- 2008 114 255
- US-A1- 2011 245 677

## Description

### Technical Field

The present invention relates to a portable ultrasonic diagnostic device and a method of operating the same.

### Background Art

Ultrasonic diagnostic devices are generally used in the medical field for obtaining information on an inside of an object to be inspected due to noninvasive and nondestructive properties thereof. Since ultrasonic diagnostic systems can provide a high-resolution image of internal organs of an object to be inspected, to a practitioner without a surgical operation of directly incising and observing the object, ultrasonic diagnostic systems are very significantly used.

An ultrasonic diagnostic device is a system which obtains images concerning tomography of soft tissue or blood flows without invasion by emitting an ultrasonic signal from a body surface of an object to be inspected toward a target part in a body and extracting information from the reflected ultrasonic signal.

Such ultrasonic diagnostic devices are generally used for diagnosing a heart, abdominal organs, urinary organs, and genital organs due to a small size, a low price, a capability of displaying in real time, and high security thereof without radiation exposure of X-rays or the like in comparison to an X-ray inspection device, a computerized tomography (CT) scanner, a magnetic resonance image (MRI) scanner, a nuclear medicine inspection device, and the like.

Recently, attempts have been made to embody a portable ultrasonic diagnostic device and connecting a portable terminal such as a smart phone and a tablet personal computer (PC) to the ultrasonic diagnostic device through wireless communications so as to perform ultrasonic diagnoses.

US 2011/245677 A1 discloses a plurality of receiving circuits and a time division unit which controls output of the reception signal to the apparatus body such that the drive signal from the apparatus body and the reception signal output to the apparatus body after being converted to a digital signal by the receiving circuit are mutually time divided and sent via the connection table.

US 2008/114255 A1 discloses a method including transmitting a non-beamformed or beamformed ultrasound wave into the medium, receiving more than one echoed ultrasound wave into digital data.

### Disclosure of Invention

### Technical Problem

In the case of a portable ultrasonic diagnostic device, during a process of obtaining ultrasonic image data, an internal signal and a high-frequency signal transmitted from the portable ultrasonic diagnostic device to a portable device have influences on each other such that signal quality suffers.

Also, since power consumption is great while transmitting data to the portable terminal, noise may occur in the internal signal due to load variation between when data is transmitted to the portable terminal and when not transmitted.

The present invention is directed to providing a portable ultrasonic diagnostic device and a method of operating the same capable of improving signal quality by preventing an internal signal and a high-frequency signal transmitted to a portable terminal from having influences on each other during a process of obtaining ultrasonic image data and capable of preventing noise occurrence in the internal signal caused by load variation between when data is transmitted to the portable terminal and when not transmitted.

### Technical Solution

In accordance with an aspect of the present invention, there is provided a portable ultrasonic diagnostic device as defined by claim 1. Further advantageous embodiments of the present invention are defined by the dependent claims.

One aspect of the present disclosure provides a portable ultrasonic diagnostic device including a transducer which generates and emits an ultrasonic pulse from an applied electrical signal toward an object to be inspected and receives an echo signal therefrom and a main circuit portion which generates an electrical pulse to be applied to the transducer, generates scan line data from the echo signal, and transmits the scan line data or frame data including a certain number of scan line data to a portable terminal. Here, the main circuit portion transmits the scan line data or the frame data during a time except a reception time of the echo signal and generates the electrical pulse during a time except a transmission time of the scan line data or the frame data.

The main circuit portion may transmit the scan line data when the reception time of the echo signal is completed, and may generate an electrical pulse for a next scan line when the scan line data is completely transmitted.

The main circuit potion may include a pulse generator which generates the electrical pulse, a beam former which generates the scan line data by beam-forming the echo signal, and a processor which transmits the scan line data. Here, the processor may transmit the scan line data when the reception time of the echo signal is completed, and the pulse generator may generate the electrical pulse for the next scan line when the scan line data is completely transmitted.

The main circuit portion may further include a buffer which stores the scan line data, and the processor may transmit the scan line data stored in the buffer when the reception time of the echo signal is completed.

The reception time of the echo signal may be determined according to a set diagnosis depth of the portable ultrasonic diagnostic device.

The main circuit portion may transmit the frame data when a reception time of an echo signal for a last scan line, which forms a frame, is completed and may generate an electrical pulse for a first scan line of a next frame when the frame data is completely transmitted.

The main circuit potion may include a pulse generator which generates the electrical pulse, a beam former which generates the scan line data by beam-forming the echo signal, a buffer which stores the generated scan line data, and a processor which transmits frame data including a certain number of the scan line data stored in the buffer. Here, the processor may transmit the frame data when the reception time of the echo signal for the last scan line is completed, and the pulse generator may generate the electrical pulse for the first scan line of the next frame when the frame data is completely transmitted.

The reception time of the echo signal may be determined according to a set diagnosis depth of the portable ultrasonic diagnostic device.

Another aspect of the present disclosure provides a method of operating a portable ultrasonic diagnostic device, the method including emitting an ultrasonic pulse toward an object to be inspected and receiving an echo signal therefrom, generating scan line data from the echo signal when a reception time of the echo signal is completed, and transmitting the scan line data to a portable terminal, and emitting an ultrasonic pulse for a next scan line when the scan line data is completely transmitted.

Still another aspect of the present disclosure provides a method of operating a portable ultrasonic diagnostic device, the method including (a) emitting an ultrasonic pulse toward an object to be inspected and receiving an echo signal, (b) generating scan line data from the echo signal when a reception time of the echo signal is completed, generating frame data including a certain number of scan line data by repeatedly performing operation (a) and operation (b), transmitting the generated frame data, and emitting an ultrasonic pulse for a first scan line of a next frame when the frame data is completely transmitted.

### Advantageous Effects

According to the present invention, a portable ultrasonic diagnostic device may improve signal quality by preventing an internal signal and a high-frequency signal transmitted to a portable terminal from having influences on each other during a process of obtaining ultrasonic image data and may prevent noise occurrence in the internal signal due to load variation between when data is transmitted to the portable terminal and when not transmitted.

### Brief Description of Drawings

FIG. 1 illustrates components of a portable ultrasonic diagnostic device according to one embodiment of the present invention.
FIG. 2 is a timing chart illustrating a process of generating an ultrasonic pulse and receiving an echo signal so as to generate scan line data.
FIG. 3 is a timing chart illustrating a process of obtaining frame data using a plurality of scan line data.
FIG. 4 is a timing chart illustrating obtainment and transmission of scan line data according to a method of transmitting scan line data.
FIG. 5 is a timing chart illustrating obtainment and transmission of frame data according to a method of transmitting frame data.
FIG. 6 is a timing chart illustrating obtainment and transmission of scan line data in the case of transmitting scan line data.
FIG. 7 is a timing chart illustrating obtainment and transmission of frame data in the case of transmitting frame data.
FIG. 8 is a flowchart illustrating a method of operating a portable ultrasonic diagnostic device according to one embodiment of the present invention.
FIG. 9 is a flowchart illustrating a method of operating a portable ultrasonic diagnostic device according to another embodiment of the present invention.

### Mode for Invention

Hereinafter, exemplary embodiments of the present invention will be described in detail with reference to the attached drawings. In the following description and the attached drawings, substantially like elements are referred to as like reference numerals such that a repetitive description will be omitted. In the description of the present invention, a detailed description on well-known functions or components of the related art will be omitted when it is deemed to obscure the essence of the present invention.

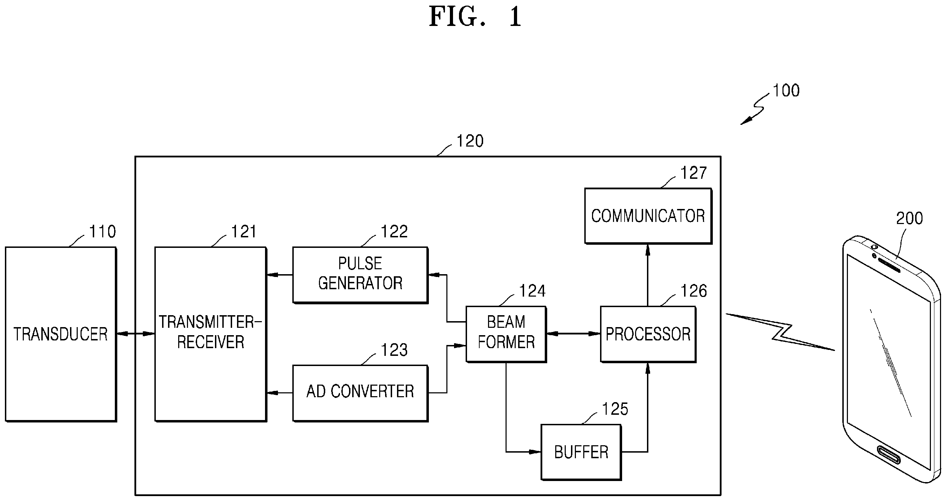

FIG. 1 illustrates components of a portable ultrasonic diagnostic device according to one embodiment of the present invention.

A portable ultrasonic diagnostic device 100 according to an embodiment of the present invention includes a transducer 110 and a main circuit portion 120.

The transducer 110 generates an ultrasonic pulse from an electrical pulse applied by the main circuit portion 120, emits the ultrasonic pulse toward an inside of an object to be inspected, converts an echo signal, which is the ultrasonic pulse reflected by the object and returns, into an electrical signal, and transmits the electrical signal to the main circuit portion 120. The transducer 110 may be formed of a piezoelectric element array module. The piezoelectric element array module may include a large number, for example, 64, 128, 192, and the like of piezoelectric elements which are arranged in an alignment shape. As the piezoelectric elements, lead zirconate titanate (PZT) having excellent electroacoustic conversion efficiency may be used. As a voltage of the electrical pulse for driving the piezoelectric elements, a voltage of+100 V to -100 V may be used.

The main circuit portion 120 generates an electrical pulse to be applied to the transducer 110, generates scan line data or frame data including a certain number scan line data by analyzing an echo signal received through the transducer 110, and transmits the scan line data or the frame data to a portable terminal 200.

The portable terminal 200 converts data received from the portable ultrasonic diagnostic device 100 into an ultrasonic image adequate for resolution of a display screen and displays the ultrasonic image through the display screen. The portable terminal 200 may be any device capable of interworking with the portable ultrasonic diagnostic device 100. For example, the portable terminal 200 may be one of a laptop personal computer (PC), a cellular phone, a portable media player, personal digital assistants (PDA), a tablet PC, a smart phone, and the like.

Data transmission and reception between portable ultrasonic diagnostic device 100 and the portable terminal 200 is performed using a wireless communication method. As the wireless communication method, Bluetooth, wireless universal serial bus (USB), wireless local area network (LAN), wireless fidelity (WiFi), Zigbee, infrared data association (IrDA), or the like may be used.

In detail, the main circuit portion 120 includes a transmitter-receiver 121, a pulse generator 122, an analog-digital (AD) converter 123, a beam former 124, a buffer 125, a processor 126, and a communicator 127.

The transmitter-receiver 121 transmits an electrical pulse generated by the pulse generator 122 to the transducer 110 and transmits an echo signal received through the transducer 110 to the AD converter 123. For example, the transmitter-receiver 121 may be configured as a switch which connects a TX circuit to the piezoelectric element array module during ultrasound transmission and connects a RX circuit to the piezoelectric element array module during echo reception.

The pulse generator 122 generates an electrical pulse to be applied to the transducer 110 to generate an ultrasonic pulse.

The AD converter 123 converts an echo signal transmitted from the transmitter-receiver 121 into a digital signal.

The beam former 124 performs TX beam forming and RX beam forming. The TX beam forming refers to allowing the pulse generator 122 to generate an adequate electrical pulse by using a parameter corresponding to the transducer 110. For example, a time of an electrical pulse is delayed according to a position of a piezoelectric element when an ultrasound is transmitted or received, so as to focus ultrasonic energy on a focal point at a certain distance. The RX beam forming refers to performing data conversion on a digital signal from the AD converter 123 in accordance with the transducer 110 and storing the data-converted digital signal in the buffer 125. For example, an electrical signal output from each piezoelectric element is time-delayed according to a position and a reception time of the piezoelectric element when an echo signal is received, and scan line data is generated by adding the time-delayed signals.

The processor 126 controls the beam former 124 to perform beam forming adequate for the transducer 110, transmits the scan line data stored in the buffer 125 to the portable terminal 200 through the communicator 127, or transmits frame data including a certain number of scan line data stored in the buffer 125 to the portable terminal 200 through the communicator 127.

Also, the processor 126 controls each element of the portable ultrasonic diagnostic device 100. The processor 126 may compress scan line data or frame data so as to reduce a bandwidth used for communication as necessary.

The communicator 127 is a communication module for transmitting or receiving data with an external display device and may use a wired or wireless communication method. As the wired communication method, a cable such as a USB cable and the like may be used. As the wireless communication method, one of Bluetooth, wireless USB, wireless LAN, WiFi, Zigbee, and IrDA may be used.

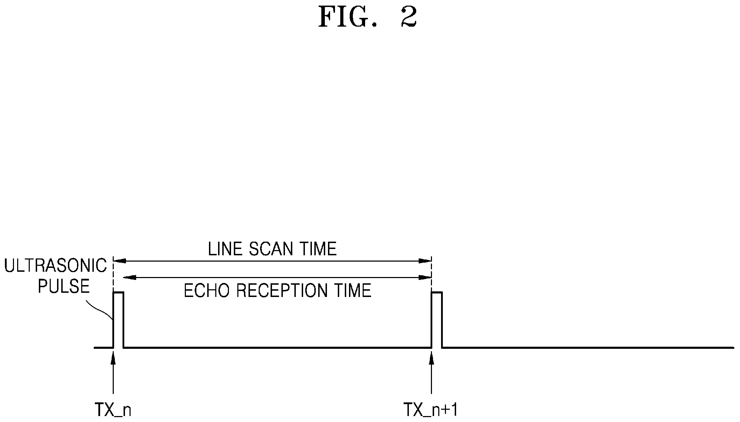

For understanding of the present invention, an operation of an existing potable ultrasonic diagnostic device (although not always a conventional technology) will be described with reference to FIGS. 2 to 5.

FIG. 2 is a timing chart illustrating a process of generating an ultrasonic pulse and receiving an echo signal so as to generate scan line data.

The portable ultrasonic diagnostic device emits an nth ultrasonic pulse. When an echo signal is received during an echo reception time and nth scan line data is generated, the portable ultrasonic diagnostic device emits an n+1th ultrasonic pulse. The echo reception time (or a line scan time) is determined according to a set diagnosis depth of the portable ultrasonic diagnostic device. For example, when an ultrasound transmission speed is 1.54 mm/µs and a diagnosis depth is set to be 10 cm, since it takes about 65 µs to transmit an ultrasonic wave to a part 10 cm deep, an echo reception time becomes 130 µs corresponding to a reciprocation time of 10 cm. That is, in the portable ultrasound diagnostic device, when a diagnosis depth is set to be 10 cm, an echo signal is received for 130 µs from when an ultrasonic pulse is emitted, which is a line scan time taken for obtaining one piece of scan line data.

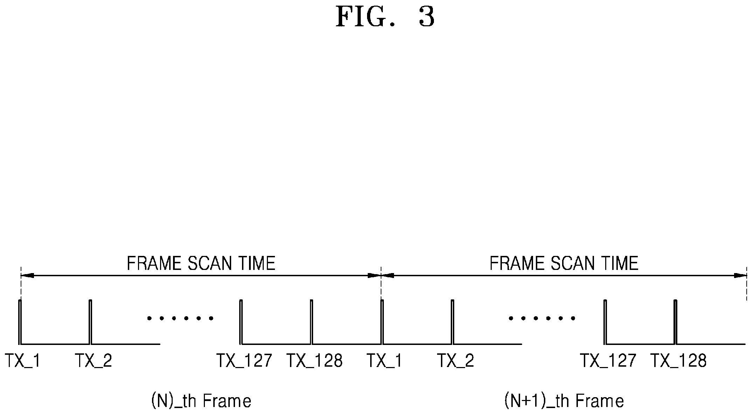

FIG. 3 is a timing chart illustrating a process of obtaining frame data using a plurality of scan line data.

For example, one frame may be formed of 128 scan lines. In this case, 128 pieces of scan line data may be generated by discharging 128 ultrasonic pulses and receiving echo signals with respect to the ultrasonic pulses so as to obtain one piece of frame data. A frame scan time, which is a time taken for obtaining one piece of frame data, is obtained by multiplying the line scan time by the number of scan lines for each frame. According to the example, 130µs×128=16.64ms.

As a method of transmitting ultrasonic image data from the portable ultrasonic diagnostic device to the portable terminal, there are a method of transmitting generated scan line data each time and a method of transmitting frame data, that is, a plurality of scan line data which form one frame.

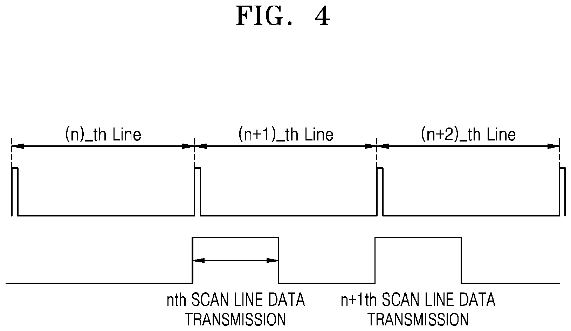

FIG. 4 is a timing chart illustrating obtainment and transmission of scan line data according to a method of transmitting scan line data.

As shown in FIG. 4, nth scan line data, which is obtained by emitting an nth ultrasonic pulse and receiving an echo signal, is transmitted to the portable terminal while an n+1th ultrasonic pulse is emitted and an echo signal thereof is received for an n+1th scan line. Accordingly, during a process of obtaining n+1th scan line data, internal signals and high frequency signals which carry the nth scan line data transmitted to the portable terminal have influences on each other such that signal quality suffers.

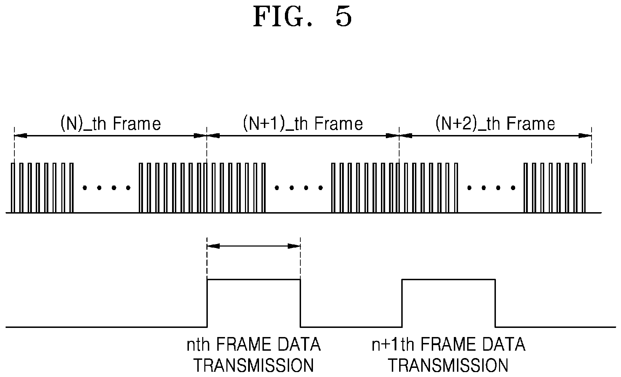

FIG. 5 is a timing chart illustrating obtainment and transmission of frame data according to a method of transmitting frame data.

As shown in FIG. 5, Nth frame data, which is obtained by emitting of ultrasonic pulses and receiving of echo signals as many as the number of scan lines per frame, is transmitted to the portable terminal. The Nth frame data is transmitted to the portable terminal while repeatedly, ultrasonic pulses are emitted and echo signals are received for an N+1th frame. Accordingly, during a process of obtaining N+1th frame data, internal signals and high frequency signals which carry the nth frame data transmitted to the portable terminal have influences on each other such that signal quality suffers.

The applicant paid attention to a possibility of allowing a reception time of an echo signal and a data transmission time to a portable terminal not to overlap with each other in consideration of a frame rate of an ultrasonic image and a time taken for obtaining scan line data or frame data.

A general frame rate of an ultrasonic image is 30 frames/second. Accordingly, a time per frame is about 33 ms. When an ultrasonic diagnosis depth is 10 cm, a line scan time is 130 µs, that is, 0.13 ms. When the number of scan lines per frame is 128, a frame scan time is 16.64 ms. When frame data is transmitted to the portable terminal, since one frame is maximally transmitted every 33 ms, a spare time of 33-16.64=16.36ms remains. When the frame rate is reduced as, for example, 15 frames/sec, a spare time further increases.

In the method of transmitting frame data, the spare time is adequate for transmitting frame data. That is, one frame is scanned for 16.64 ms and then a spare time of maximum 16.36 ms is available before starting scanning of a next frame. Here, it is only needed to transmit frame data during the spare time.

In the method of transmitting scan line data, a maximum allowed period of transmitting scan line data is about 260 µs when a frame rate is 30 frames/sec and the number of scan lines per frame is 128. Accordingly, when an ultrasonic diagnosis depth is 10 cm, one scan line is scanned for 130 µs and then a spare time of maximum 130 µs is available before starting scanning of a next scan line. Here, it is only needed to transmit scan line data during the spare time. The spare time is also adequate for transmitting scan line data.

On the basis of the above point, the main circuit portion 120, during a time except a reception time of an echo signal, may transmit scan line data or frame data to the portable terminal 200, and during a time except scan line data or frame data transmission time, may generate and apply an electrical pulse for an ultrasonic pulse to the transducer 110 and may receive an echo signal therefrom.

As one embodiment, in the case of a method of transmitting scan line data from the portable ultrasonic diagnostic device 100 to the portable terminal 200, when the reception time of the echo signal is completed, the main circuit portion 120 may transmit corresponding scan line data, and when the scan line data is completely transmitted, the main circuit portion 120 may generate and apply an electrical pulse for a next scan line to the transducer 110.

In detail, the processor 126 may transmit scan line data stored in the buffer 125 when the reception time of the echo signal is completed, and the pulse generator 122 may generate and apply an electrical pulse for a next scan line to the transducer 110 through the transmitter-receiver 121 when the scan line data is completely transmitted.

For this, when an ultrasonic pulse is emitted and the reception time of the echo signal is completed therefrom, the beam former 124 may transmit a signal, which indicates the completion, to the processor 126, and the processor 126 may transmit scan line data stored in the buffer 125 to the portable terminal 200 through the communicator 127 in response to the signal.

Also, when the scan line data is completely transmitted, the processor 126 may transmit a signal, which indicates the completion, to the beam former 124 and/or the pulse generator 122, and the beam former 124 and the pulse generator 122 may generate and apply an electrical pulse for a next scan line to the transducer 110 through the transmitter-receiver 121.

The signal, which is transmitted between the processor 126 and the beam former 124 and/or the pulse generator 122, may be an actual electrical signal or may be a particular register value of a central processing unit (CPU) or a field-programmable gate array (FPGA).

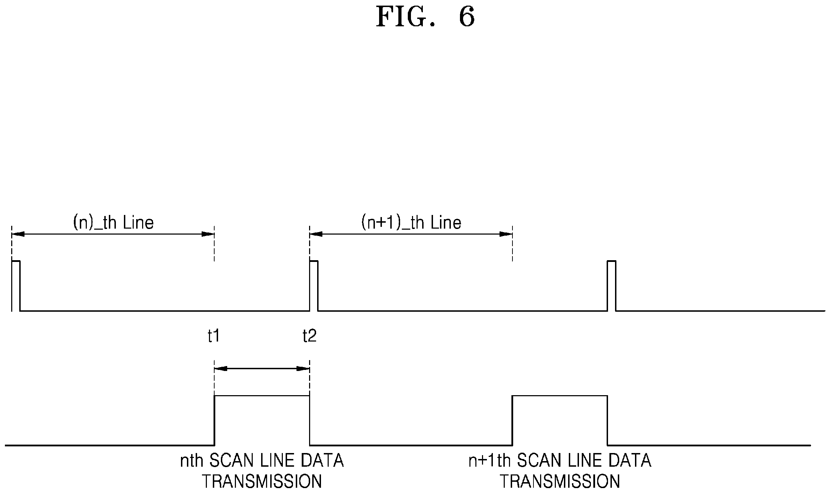

FIG. 6 is a timing chart illustrating obtainment and transmission of scan line data according to an embodiment of the present invention in the case of a method of transmitting scan line data from the portable ultrasonic diagnostic device 100 to the portable terminal 200.

As shown in FIG. 6, when an ultrasonic pulse for an nth scan line is emitted and a reception time of an echo signal is completed therefrom (t1), the processor 126 transmits nth scan line data in response to a signal from the beam former 124. Also, when the nth scan line data is completely transmitted (t2), the beam former 124 and the pulse generator 122 generates and applies an electrical pulse for an n+1th scan line to the transducer 110 in response to a signal from the processor 126.

As another embodiment, in the case of a method of transmitting frame data from the portable ultrasonic diagnostic device 100 to the portable terminal 200, when a reception time of an echo signal for a last scan line, which forms a frame, is completed, the main circuit portion 120 may transmit corresponding frame data, and when the frame data is completely transmitted, the main circuit portion 120 may generate and apply an electrical pulse for a first scan line of a next frame to the transducer 110.

In detail, the processor 126 may transmit corresponding frame stored in the buffer 125 when the reception time of the echo signal for the last scan line, which forms the frame, is completed, and the pulse generator 122 may generate and apply an electrical pulse for the first scan line of the next frame to the transducer 110 through the transmitter-receiver 121 when the frame data is completely transmitted.

For this, when an ultrasonic pulse for the last scan line, which forms the frame, is emitted and the reception time of the echo signal is completed therefrom, the beam former 124 may transmit a signal, which indicates the completion, to the processor 126, and the processor 126 may transmit frame data stored in the buffer 125 to the portable terminal 200 through the communicator 127 in response to the signal.

Also, when the frame data is completely transmitted, the processor 126 may transmit a signal, which indicates the completion, to the beam former 124 and/or the pulse generator 122, and the beam former 124 and the pulse generator 122 may generate and apply an electrical pulse for a first scan line of a next frame to the transducer 110 through the transmitter-receiver 121.

The signal, which is transmitted between the processor 126 and the beam former 124 and/or the pulse generator 122, may be an actual electrical signal or may be a particular register value of a CPU or an FPGA.

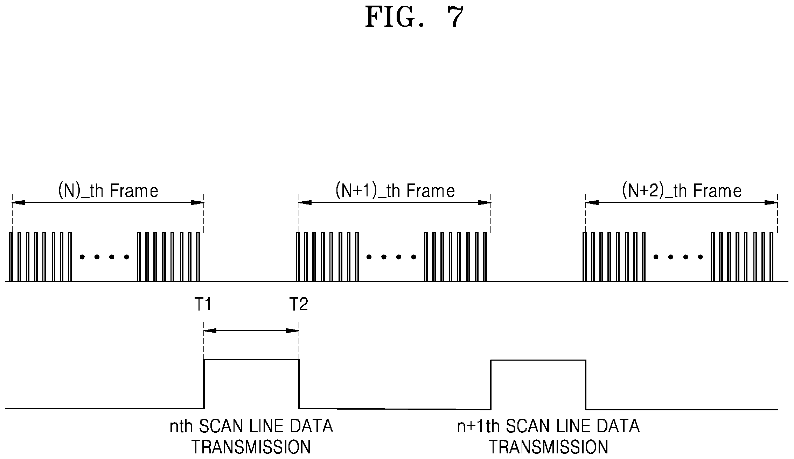

FIG. 7 is a timing chart illustrating obtainment and transmission of frame data according to an embodiment of the present invention in the case of a method of transmitting frame data from the portable ultrasonic diagnostic device 100 to the portable terminal 200.

As shown in FIG. 7, when an ultrasonic pulse for a last scan line, which forms an Nth frame, is emitted and a reception time of an echo signal is completed therefrom (T1), the processor 126 transmits Nth frame data stored in the buffer 125 in response to the signal from the beam former 124. Also, when the Nth frame data is completely transmitted (T2), the beam former 124 and the pulse generator 122 generates and applies an electrical pulse for a first scan line of an N+1th frame to the transducer 110 in response to a signal from the processor 126.

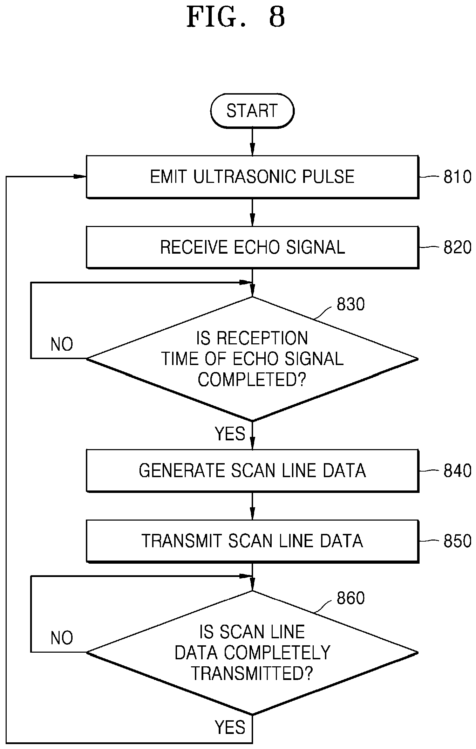

FIG. 8 is a flowchart illustrating a method of operating a portable diagnostic device according to one embodiment of the present invention and illustrates operations of a method of transmitting scan line data from the portable ultrasonic diagnostic device to a portable device.

The portable ultrasonic diagnostic device 100 emits an ultrasonic pulse toward an object to be inspected (810) and receives an echo signal which is reflected and returns (820).

When the ultrasonic pulse is emitted and a reception time of the echo signal is completed therefrom (830), the portable ultrasonic diagnostic device 100 generates scan line data (840) and transmits the scan line data to the portable terminal 200 (850).

When the scan line data is completely transmitted (860), the portable ultrasonic diagnostic device 100 returns to operation 810 to emit an ultrasonic pulse for a next scan line and performs the operations next thereto.

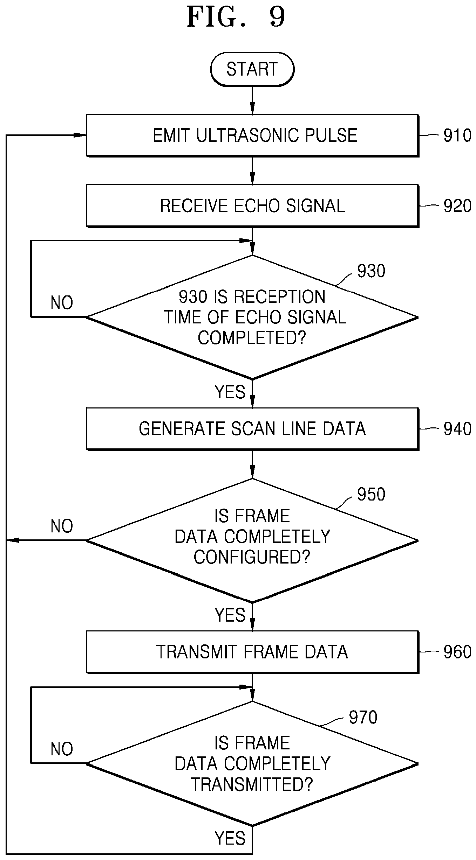

FIG. 9 is a flowchart illustrating a method of operating a portable diagnostic device according to another embodiment of the present invention and illustrates operations of a method of transmitting frame data from the portable ultrasonic diagnostic device to a portable device.

The portable ultrasonic diagnostic device 100 emits an ultrasonic pulse toward an object to be inspected (910) and receives an echo signal which is reflected and returns (920).

When the ultrasonic pulse is emitted and a reception time of the echo signal is completed therefrom (930), the portable ultrasonic diagnostic device 100 generates scan line data (940).

When frame data is not completely configured (950), the portable ultrasonic diagnostic device 100 repeatedly performs operation 910 to operation 940.

When the frame data is completely configured (950), that is, when an ultrasonic pulse for a last scan line, which forms a frame, is emitted and a reception time of an echo signal is completed therefrom such that scan line data is generated, the portable ultrasonic diagnostic device 100 transmits corresponding frame data (960).

When the frame data is completely transmitted (970), the portable ultrasonic diagnostic device 100 returns to operation 910 to emit an ultrasonic pulse for a first scan line of a next frame and performs the operations next thereto.

The exemplary embodiments have been described above. It will be understood by one of ordinary skill in the art that modifications may be made without departing from the essential features of the present invention. Therefore, the disclosed embodiments should be considered in a descriptive point of view not in limitative one. The scope of the present invention is defined by the claims not by the above description, and it should be understood that all differences within the equivalent scope thereof are included in the present invention.

## Claims

1. A portable ultrasonic diagnostic device (100) comprising:
a transducer (110) which generates and emits an ultrasonic pulse from an applied electrical pulse toward an object to be inspected and receives an echo signal therefrom; and
a main circuit portion (120) which generates an electrical pulse to be applied to the transducer, generates scan line data from the echo signal, and transmits frame data including a certain number of scan line data to a portable terminal (200),
**characterized in that:**
the main circuit portion (120) transmits, using a wireless communication method, the scan line data or the frame data during a time except a reception time of the echo signal and generates the electrical pulse during a time except a transmission time of the frame data,
wherein the main circuit portion (120) transmits the frame data when a reception time of an echo signal for the last scan line, which forms a frame, is completed, and generates an electrical pulse for the first scan line of the next frame when the frame data is completely transmitted, and
wherein the main circuit potion (120) comprises:
a pulse generator (122) which generates the electrical pulse;
a beam former (124) which generates the scan line data by beam-forming the echo signal;
a buffer (125) which stores the generated scan line data; and
a processor (126) which transmits frame data including a certain number of the scan line data stored in the buffer,
wherein the processor (126) transmits the frame data when the reception time of the echo signal for the last scan line is completed, and
wherein the pulse generator (122) generates the electrical pulse for the first scan line of the next frame when the frame data is completely transmitted.

2. The portable ultrasonic diagnostic device of claim 1, wherein the reception time of the echo signal is determined according to a set diagnosis depth of the portable ultrasonic diagnostic device.

3. A method of operating a portable ultrasonic diagnostic device according to claim 1 or 2, comprising:
(a) emitting an ultrasonic pulse toward an object to be inspected and receiving an echo signal;
(b) generating scan line data from the echo signal when a reception time of the echo signal is completed;
generating frame data including a certain number of scan line data by repeatedly performing operation (a) and operation (b);
transmitting the generated frame data; and
emitting an ultrasonic pulse for the first scan line of the next frame when the frame data is completely transmitted.

## Patentansprüche

1. Tragbare Ultraschall-Diagnosevorrichtung (100), umfassend:
einem Wandler (110), der einen Ultraschallimpuls aus einem angelegten elektrischen Impuls erzeugt und in Richtung eines zu untersuchenden Objekts aussendet und ein Echosignal davon empfängt; und
einen Hauptstromkreisabschnitt (120), der einen an den Wandler anzulegenden elektrischen Impuls erzeugt, Abtastzeilendaten aus dem Echosignal erzeugt und Frame-Daten, die eine bestimmte Anzahl von Abtastzeilendaten einschließen, an ein tragbares Endgerät (200) überträgt, **dadurch gekennzeichnet, dass:**
der Hauptstromkreisabschnitt (120) unter Verwendung eines drahtlosen Kommunikationsverfahrens die Abtastzeilendaten oder die Frame-Daten während einer Zeit außer einer Empfangszeit des Echosignals sendet und den elektrischen Impuls während einer Zeit außer einer Sendezeit der Frame-Daten erzeugt,
wobei der Hauptstromkreisabschnitt (120) die Frame-Daten sendet, wenn eine Empfangszeit eines Echosignals für die letzte Abtastzeile, die einen Frame bildet, abgeschlossen ist, und einen elektrischen Impuls für die erste Abtastzeile des nächsten Frame erzeugt, wenn die Frame-Daten vollständig gesendet sind, und
wobei der Hauptstromkreisabschnitt (120) umfasst:
einen Impulsgeber (122), der den elektrischen Impuls erzeugt;
einen Strahlformer (124), der die Abtastzeilendaten durch Strahlformung des Echosignals erzeugt;
einen Puffer (125), der die erzeugten Abtastzeilendaten speichert; und
einen Prozessor (126), der Frame-Daten überträgt, die eine bestimmte Anzahl der im Puffer gespeicherten Abtastzeilendaten einschließen,
wobei der Prozessor (126) die Frame-Daten sendet, wenn die Empfangszeit des Echosignals für die letzte Abtastzeile abgeschlossen ist, und
wobei der Impulsgeber (122) den elektrischen Impuls für die erste Abtastzeile des nächsten Frame erzeugt, wenn die Frame-Daten vollständig übertragen sind.

2. Tragbare Ultraschall-Diagnosevorrichtung nach Anspruch 1, wobei die Empfangszeit des Echosignals entsprechend einer eingestellten Diagnosetiefe der tragbaren Ultraschall-Diagnosevorrichtung bestimmt wird.

3. Verfahren zum Betreiben einer tragbaren Ultraschall-Diagnosevorrichtung nach Anspruch 1 oder 2, umfassend:
(a) Aussenden eines Ultraschallimpulses in Richtung eines zu untersuchenden Objekts und Empfangen eines Echosignals;
(b) Erzeugen von Abtastzeilendaten aus dem Echosignal, wenn eine Empfangszeit des Echosignals abgeschlossen ist;
Erzeugen von Frame-Daten, die eine bestimmte Anzahl von Abtastzeilendaten enthalten, durch wiederholtes Ausführen von Operation (a) und Operation (b);
Senden der erzeugten Frame-Daten; und
Aussenden eines Ultraschallimpulses für die erste Abtastzeile des nächsten Frame, wenn die Frame-Daten vollständig übertragen sind.

## Revendications

1. Dispositif de diagnostic ultrasonore portatif (100) comprenant :
un transducteur (110) qui génère et émet une impulsion ultrasonore à partir d'une impulsion électrique appliquée en direction d'un objet à inspecter et reçoit un signal d'écho de celui-ci ; et
une partie circuit principal (120) qui génère une impulsion électrique à appliquer au transducteur, génère des données de ligne de balayage à partir du signal d'éco, et transmet des données de trame comprenant un certain nombre de données de ligne de balayage à un terminal portatif (200), **caractérisé en ce que :**
la partie circuit principal (120) transmet, en utilisant un procédé de communication sans fil, les données de ligne de balayage ou les données de trame pendant un temps à l'exception d'un temps de réception du signal d'écho et génère l'impulsion électrique pendant un temps à l'exception d'un temps de transmission des données de trame,
dans lequel la partie circuit principal (120) transmet les données de trame lorsqu'un temps de réception d'un signal d'écho pour la dernière ligne de balayage, qui forme une trame, est terminé, et génère une impulsion électrique pour la première ligne de balayage de la trame suivante lorsque les données de trame sont complètement transmises, et
dans lequel la partie circuit principal (120) comprend :
un générateur d'impulsion (122) qui génère l'impulsion électrique ;
un formeur de faisceau (124) qui génère les données de ligne de balayage par formation de faisceau du signal d'écho ;
un tampon (125) qui stocke les données de ligne de balayage générées ; et
un processeur (126) qui transmet des données de trame comprenant un certain nombre des données de ligne de balayage stockées dans le tampon,
dans lequel le processeur (126) transmet les données de trame lorsque le temps de réception du signal d'écho pour la dernière ligne de balayage est terminé, et
dans lequel le générateur d'impulsion (122) génère l'impulsion électrique pour la première ligne de balayage de la trame suivante lorsque les données de trame sont complètement transmises.

2. Dispositif de diagnostic ultrasonore portatif selon la revendication 1, dans lequel le temps de réception du signal d'écho est déterminé selon une profondeur de diagnostic définie du dispositif de diagnostic ultrasonore portatif.

3. Procédé d'utilisation d'un dispositif de diagnostic ultrasonore portatif selon la revendication 1 ou 2, comprenant :
(a) l'émission d'une impulsion ultrasonore en direction d'un objet à inspecter et la réception d'un signal d'écho ;
(b) la génération de données de ligne de balayage du signal d'écho lorsqu'un temps de réception du signal d'écho est terminé ;
la génération de données de trame comprenant un certain nombre de données de ligne de balayage par réalisation répétée de l'opération (a) et de l'opération (b) ;
la transmission des données de trame générées ; et
l'émission d'une impulsion ultrasonore pour la première ligne de balayage de la trame suivante lorsque les données de trame sont complètement transmises.
